(19) Europäisches Patentamt
European Patent Office
Office européen des brevets

(11) **EP 3 683 360 B1**

(12) **FASCICULE DE BREVET EUROPEEN**

(45) Date de publication et mention
de la délivrance du brevet:
**03.11.2021 Bulletin 2021/44**

(51) Int Cl.:
***E02D 1/02*** *(2006.01)* ***G01N 33/24*** *(2006.01)*

(21) Numéro de dépôt: **20152118.4**

(22) Date de dépôt: **16.01.2020**

(54) **PENETROMETRE STATIQUE A SYSTEME COMPRESSIBLE DEPORTE ET UTILISATION D'UN TEL PENETROMETRE**

STATISCHER PENETROMETER MIT AUSGELAGERTEM KOMPRIMIERBAREN SYSTEM, UND VERWENDUNG EINES SOLCHEN PENETROMETERS

STATIC PENETROMETER WITH OFFSET COMPRESSING SYSTEM AND USE OF SUCH A PENETROMETER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.01.2019 FR 1900439**

(43) Date de publication de la demande:
**22.07.2020 Bulletin 2020/30**

(73) Titulaire: **Equatech.R&D**
**74960 Annecy (FR)**

(72) Inventeurs:
• **RIEGEL, Pierre**
**74330 POISY (FR)**
• **NOGUEIRA GONCALVES, Carlos**
**73400 Ugine (FR)**

(74) Mandataire: **IP Trust**
**2, rue de Clichy**
**75009 Paris (FR)**

(56) Documents cités:
BE-A- 829 984      FR-A1- 2 584 186
SU-A1- 596 861      SU-A1- 718 762

**EP 3 683 360 B1**

Printed by Jouve, 75001 PARIS (FR)

**Description**

**DOMAINE DE L'INVENTION**

La présente invention concerne le domaine de la géotechnique et de la géologie. Elle concerne en particulier un dispositif de mesure de la résistance à la pénétration d'un sol, appelé communément un pénétromètre, et des procédés de mesure associés.

**ARRIERE PLAN TECHNOLOGIQUE DE L'INVENTION**

La compacité des sols est habituellement mesurée, soit en mode statique, soit en mode dynamique, par un dispositif de mesure appelé pénétromètre. Un pénétromètre comporte classiquement des tiges raccordées bout à bout pour former un train de tiges au bout duquel est fixée une pointe de mesure, destinée à s'enfoncer dans le sol sur des profondeurs pouvant atteindre plusieurs dizaines de mètres.

En mode statique, le train de tiges est poussé par des vérins, provoquant l'enfoncement progressif de la pointe de mesure ; cette dernière mesure la résistance de pointe et éventuellement le frottement latéral sur un manchon cylindrique situé au-dessus de la pointe. Ces mesures sont enregistrées de manière continue ou discontinue selon un pas régulier. La mesure statique de la résistance à la pénétration du sol est sans conteste la plus précise car elle est réalisée directement sur la pointe de mesure, au fond du sondage.

Ce mode de mesure statique se heurte néanmoins à quelques difficultés. Lorsque la résistance du sol est importante (sols très compacts), il devient très complexe d'appliquer une poussée suffisante pour générer l'enfoncement de la pointe. Par ailleurs, quand la pointe de mesure et le train de tiges sont enfoncés à plusieurs mètres, le frottement exercé par le sol sur le train de tiges doit également être vaincu par une forte poussée pour poursuivre l'enfoncement. Il en résulte que le poids, la puissance et la qualité de l'ancrage des machines qui actionnent les vérins ne sont parfois pas suffisants pour assurer en toutes circonstances une mesure en mode statique.

Il existe par ailleurs un autre mode de mesure dit mode dynamique basé sur l'enfoncement de la pointe par battage du train de tiges. Le battage est réalisé en faisant chuter une masse ou un marteau sur une enclume enfonçant le train de tiges par à-coups. Connaissant l'énergie libérée par la chute de la masse et connaissant l'enfoncement par à-coup, on calcule la résistance à la pénétration du sol, compte tenu de divers coefficients de perte d'énergie. Ce mode dynamique présente une plus grande capacité de pénétration que le mode statique, en particulier dans les sols de compacité élevée. Une mesure en mode dynamique est néanmoins beaucoup moins précise qu'une mesure en mode statique et ne donne qu'une information partielle de la résistance du sol en fonction de la profondeur, du fait de l'enfoncement par à-coup et en général de la non dissociation de la résistance de pointe par rapport aux frottements latéraux parasites.

Le document FR2584186 décrit un dispositif de mesure des caractéristiques des sols par pénétration dynamique comprenant un train de tiges pleines se terminant par une pointe et coulissant à l'intérieur d'un train de tiges creuses, et une tête de battage rigidement solidaire des tiges creuses et élastiquement solidaire des tiges pleines par interposition d'un ressort. La chute d'un mouton sur la tête de battage provoque l'enfoncement des trains de tiges. Pendant le relevage et la nouvelle chute du mouton (durée 0,3 à 5 secondes), le ressort se détend et la pointe continue à s'enfoncer jusqu'à ce que la réaction du sol soit égale à l'effort de compression du ressort. Ce dispositif présente l'inconvénient de ne faire, entre deux enfoncements dynamiques par chute d'un mouton sur la tête de battage, que des mesures ponctuelles et discontinues en mode statique, à des profondeurs aléatoires dépendant de la résistance du sol.

D'autres pénétromètres dits statiques dynamiques, sont capables de travailler en mode mixte dynamique et statique, dès que le mode tout statique n'est plus possible, typiquement en présence de couches de sol présentant une compacité élevée, la résistance à la pénétration devenant trop importante.

On connaît notamment le document EP2535460, qui décrit un dispositif comprenant une tige de mesure terminée par une pointe de mesure, un tube de protection disposé autour de la tige de mesure, des moyens de descente et de relevage pour effectuer l'enfoncement dans le sol en mode statique, des moyens de frappe pour effectuer l'enfoncement dans le sol en mode dynamique et des moyens de mesure pour déterminer la résistance à la pénétration du sol. La construction particulière de ce dispositif permet d'isoler l'enfoncement dynamique du tube de protection extérieur, de la prise de mesure statique de la résistance en pointe et du frottement latéral à travers la tige de mesure intérieure. Ainsi la tige de mesure ne subit pas les efforts mécaniques exercés par les moyens de frappe et la prise de mesure peut être réalisée en mode statique.

La structure de ce dispositif reste néanmoins complexe et lourde à mettre en œuvre, tant du point de vue du dispositif lui-même que des équipements connexes nécessaires pour déplacer et utiliser ledit dispositif. De plus, la phase de battage ne permet pas la mesure de la résistance en pointe (celle-ci n'étant pas dissociée des frottements latéraux du manchon), et ce à l'identique du dispositif décrit dans le document FR2584186, de sorte que certaines particularités importantes du terrain peuvent échapper à la connaissance du géologue.

**OBJET DE L'INVENTION**

Un objet de la présente invention est de proposer une solution alternative aux solutions de l'état de l'art, en particulier un pénétromètre statique simple de mise en œuvre, robuste et permettant des mesures précises et continues de la compacité d'un sol.

## BREVE DESCRIPTION DE L'INVENTION

L'invention concerne un pénétromètre selon la revendication indépendante 1 comprenant:

- Au moins une tige centrale terminée à une première extrémité par une pointe de mesure ;
- Au moins un tube creux entourant la tige centrale, cette dernière étant apte à coulisser à l'intérieur du tube creux ;
- Une cellule de mesure en contact avec le tube creux, destinée à transmettre une force appliquée par des moyens d'appui, de manière à provoquer un enfoncement en mode statique dans le sol du tube creux et de la tige centrale ;

Le pénétromètre comprend un système compressible assurant une liaison élastique entre la cellule de mesure et une seconde extrémité de la tige. Le pénétromètre est remarquable en ce que ledit système compressible comporte:

- Une première chambre à huile formée dans la cellule de mesure, et un premier piston solidaire d'une seconde extrémité de la tige centrale et apte à coulisser dans la première chambre ;
- Au moins une colonne déportée par rapport à l'axe de la tige centrale, qui comprend une deuxième chambre à huile en communication fluidique avec la première chambre, un deuxième piston apte à coulisser dans la deuxième chambre et un dispositif compressible étalonné en contact avec le deuxième piston ;
- Un capteur de pression relié à la deuxième chambre et/ou un capteur de déplacement apte à mesurer la longueur du dispositif compressible étalonné.

D'autres caractéristiques avantageuses de l'invention sont:

- le capteur de pression est apte à mesurer en continu la pression dans la deuxième chambre, au cours de l'enfoncement ;
- le capteur de déplacement est apte à mesurer en continu la longueur du dispositif compressible étalonné, au cours de l'enfoncement ;
- le pénétromètre peut comprendre un contrôleur électronique configuré pour enregistrer les mesures issues du ou des capteurs et pour activer ou désactiver les moyens supplémentaires d'enfoncement. Les revendications dépendantes 2-12 montrent aussi d'autres caractéristiques avantageuses de l'invention.

L'invention concerne également différentes utilisations du pénétromètre tel que ci-dessus :

- une utilisation pour la mesure de la résistance à la pénétration d'un sol,
- une utilisation pour l'évaluation du caractère liquéfiable d'un sol,
- une utilisation pour la mesure des propriétés élastoplastiques d'un sol.

## BREVE DESCRIPTION DES FIGURES

D'autres caractéristiques et avantages de l'invention ressortiront de la description détaillée de l'invention qui va suivre en référence aux figures annexées sur lesquelles :

[Fig. 1] La figure 1 présente un schéma de principe d'un pénétromètre selon un premier mode de réalisation de l'invention ;

[Fig. 2] La figure 2 présente un schéma de principe d'un pénétromètre selon un deuxième mode de réalisation de l'invention.

## DESCRIPTION DETAILLEE DE L'INVENTION

Dans la partie descriptive, les mêmes références sur les figures pourront être utilisées pour des éléments de même type. Les figures sont des représentations schématiques qui, dans un objectif de lisibilité, ne sont pas nécessairement à l'échelle.

La présente invention concerne un pénétromètre 100 pouvant être notamment utilisé pour la mesure en mode statique de la résistance à la pénétration d'un sol.

Le pénétromètre 100 comprend au moins une tige centrale 1 terminée à une première extrémité par une pointe de mesure 11. A titre d'exemple, une pointe de mesure bien connue de type « Gouda » pourra être montée au niveau de la première extrémité de ladite tige centrale 1.

Le pénétromètre 100 comprend au moins un tube creux 2 entourant la tige centrale 1. Les diamètres respectifs du tube creux 2 et de la tige centrale 1 sont adaptés de sorte que cette dernière puisse coulisser librement à l'intérieur du tube creux 2.

Le couple formé par la tige centrale 1 et le tube creux 2 est destiné à s'enfoncer dans le sol, la pointe 11 en bout ayant pour fonction de mesurer la résistance à la pénétration du sol. Pour tester la résistance du sol à des profondeurs diverses, des tiges 1 et tubes 2 additionnels peuvent être raccordés bout à bout, pour former un train de couples tige/tube, pouvant être enfoncé dans le sol sur des profondeurs de plusieurs dizaines de mètres.

Le pénétromètre 100 comprend également une cellule de mesure 3 en contact avec le tube creux 2. La cellule de mesure 3 est destinée à transmettre une force F appliquée par des moyens d'appui, de manière à provoquer un enfoncement en mode statique dans le sol du couple formé par le tube creux 2 et la tige centrale 1. Les moyens d'appui (non représentés) susceptibles d'appliquer ladite force F seront décrits plus loin.

La cellule de mesure 3 pourra dans certains cas être solidaire du tube creux 2, notamment si on utilise le frottement du tube creux 2 dans le sol comme élément de réaction. Dans les cas où la cellule de mesure 3 est seulement en contact avec le tube creux 2, c'est la poussée par les moyens d'appui qui maintient la continuité mécanique entre cellule 3 et tube 2. Un adaptateur en tête de tube creux 2 peut permettre le passage indifférent d'un mode à l'autre.

Le pénétromètre 100 comprend en outre, un système compressible 6 assurant une liaison élastique entre la cellule de mesure 3 et une seconde extrémité 12 de la tige centrale 1 (ou du train de tiges centrales). La seconde extrémité 12 se trouve à l'opposé de la première extrémité qui supporte la pointe de mesure 11.

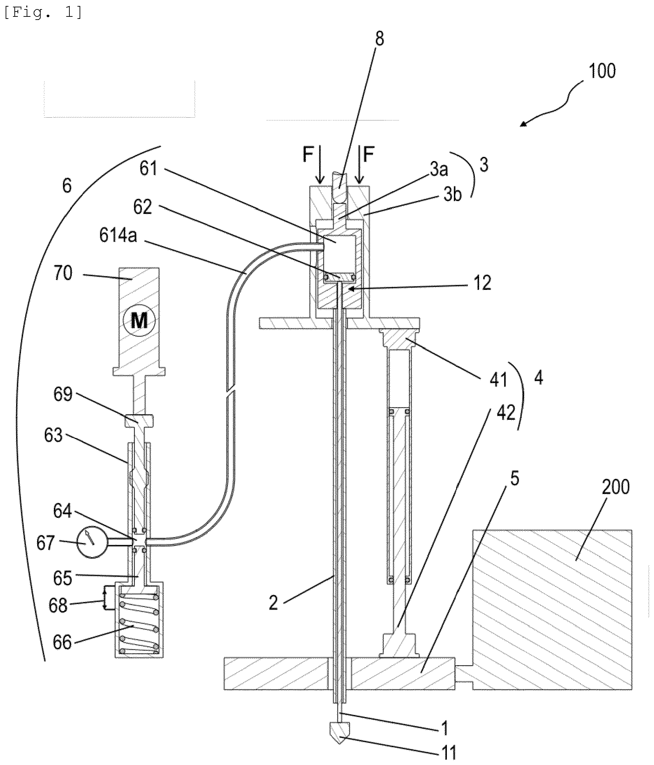

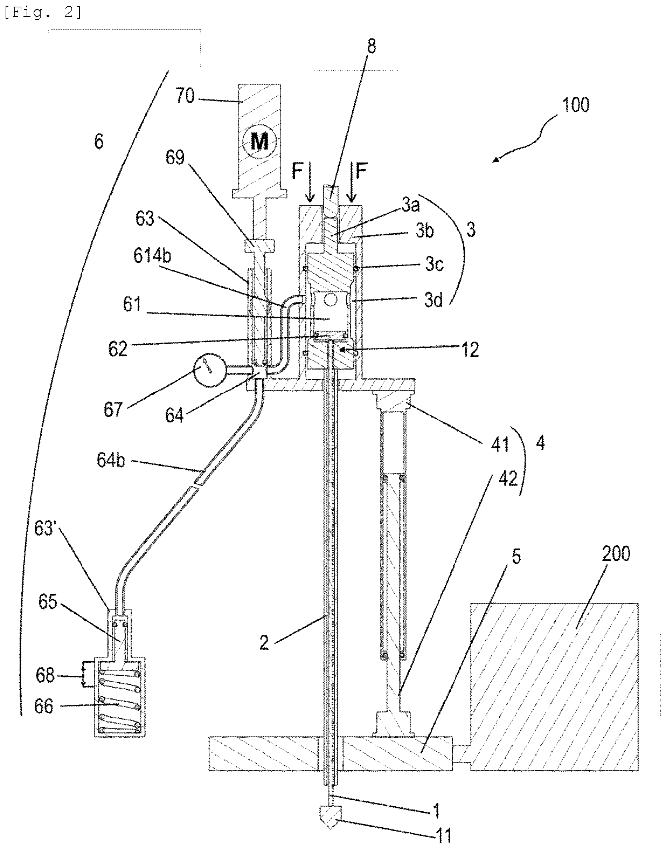

Comme illustré sur les figures 1 et 2, le système compressible 6 comporte une première chambre à huile 61 formée dans la cellule de mesure 3, et un premier piston 62 solidaire de la seconde extrémité 12 de la tige centrale 1 ; le premier piston 62 est apte à coulisser dans la première chambre 61. En particulier, lorsque la pointe de mesure 11 est totalement sortie par rapport au tube creux 2, le premier piston 62 sera en position basse (selon la disposition des figures 1 et 2) dans la première chambre 61 ; lorsque la pointe de mesure 11 est effacée, c'est-à-dire en appui contre le tube creux 2, le premier piston 62 sera dans une position plus haute dans la première chambre 61. Le premier piston 62 est donc apte à coulisser dans la première chambre 61 en fonction de la force de réaction que le sol applique sur la pointe de mesure 11.

Le système compressible 6 comporte également une colonne 63 déportée par rapport à l'axe de la tige centrale 1. La colonne déportée 63 comporte une deuxième chambre à huile 64 en communication fluidique avec la première chambre 61 et un deuxième piston 65 apte à coulisser dans la deuxième chambre 64. Le système compressible 6 comporte également un dispositif compressible étalonné 66 en contact avec le deuxième piston 65.

Selon un premier mode de réalisation illustré sur la figure 1, la communication fluidique entre la première chambre 61 et la deuxième chambre 64 est faite au moyen d'un tuyau flexible 614a, qui autorise le déport à une distance choisie de la colonne 63 par rapport à l'ensemble cellule de mesure 3 / train de tube 2 & tige 1 et à l'axe d'enfoncement.

Selon un deuxième mode de réalisation illustré sur la figure 2, la communication fluidique entre la première chambre 61 et la deuxième chambre 64 est faite au moyen d'un conduit rigide 614b et relativement court, qui permet également un déport de la colonne 63 par rapport à l'ensemble cellule de mesure 3 / train de tube 2 et tige 1, mais plus limité que dans le premier mode de réalisation. Le dispositif compressible 66 pouvant être un élément encombrant de la colonne 63, il est avantageusement déporté par rapport à la colonne 63, dans une deuxième colonne 63' : la deuxième chambre 64 est alors prolongée par un deuxième conduit 64b, avantageusement flexible et de longueur appropriée, jusqu'au piston 65 disposé dans la deuxième colonne 63', en contact avec le dispositif compressible 66.

Nous verrons plus tard que le premier ou le deuxième mode de mise en œuvre peut être plus avantageux selon le type d'essai à réaliser.

Le déplacement du premier piston 62 dans la première chambre 61 en fonction de la force de réaction que le sol applique sur la pointe de mesure 11, va être transmis au deuxième piston 65 dans la deuxième chambre 64, du fait de la faible compressibilité de l'huile présente dans et entre les deux chambres. Le deuxième piston 65 va alors appliquer une charge sur le dispositif compressible étalonné 66, susceptible de comprimer ou de laisser s'allonger ledit dispositif 66.

Dans le système compressible 6 du pénétromètre 100 selon l'invention, la longueur du dispositif compressible étalonné 66 pourra avantageusement varier de 40 à 100mm entre son état allongé et son état complètement rétracté. Notons que la raideur du dispositif compressible 66 peut être adaptée à la dureté du sol envisagé d'après enquête préalable. En particulier, on pourra choisir la raideur du dispositif compressible 66 de sorte qu'il passe de son état allongé (pour une charge nulle) à son état complètement rétracté pour une charge appliquée comprise entre 100 et 500 bars.

Selon une première variante, le dispositif compressible 66 est constitué par un ressort étalonné. La longueur du ressort varie linéairement avec la charge qui lui est appliquée.

Selon une deuxième variante, le dispositif compressible étalonné 66 est constitué d'une pluralité de rondelles Belleville, montées en opposition. La déformation d'un tel assemblage est quasiment linéaire, et sa raideur peut être définie comme : $k = n \times P/_{h_0}$, où n est le nombre de rondelles, P est la charge d'aplatissement d'une rondelle, au-delà de laquelle la rondelle ne se déforme pratiquement plus et ho est la flèche maximale. Cette variante est avantageuse en ce que la raideur de l'assemblage de rondelles peut être facilement ajustée par l'ajout, ou le retrait de rondelles, lesquelles peuvent être assemblées dans une grande variété de dispositions : en opposition ou en série ou encore en une combinaison de ces dispositions, et en proportion variable. L'utilisation d'une pluralité de rondelles Belleville est particulièrement avantageuse en ce qu'elle autorise un encombrement vertical, dans la colonne déportée 63, plus réduit que lorsqu'un ressort est utilisé. De manière identique à la première variante, la charge appliquée à la pluralité de rondelles Belleville est également liée à la longueur du dispositif compressible, correspondant à l'aplatissement (rétractation) et/ou l'expansion (allongement) de l'assemblage de rondelles.

Le dispositif compressible étalonné n'est bien-sûr pas limité aux deux variantes évoquées ci-dessus et pourrait alternativement être formé par un ressort à gaz ou tout élément élastique (élastomère, etc), permettant une rétractation et un allongement de l'ordre de grandeur cité plus haut, pour des charges appliquées telles qu'énoncées précédemment.

Le système compressible 6 selon l'invention comporte en outre un capteur de pression 67 relié à la deuxième chambre 64 apte à mesurer la pression dans ladite deuxième chambre 64. En tenant compte des rapports de surface entre la pointe 11, et les premier 62 et deuxième 65 pistons, on peut ainsi extraire à partir de la mesure faite par le capteur de pression 67, la pression s'exerçant au niveau de la pointe 11, représentative de la résistance du sol.

Alternativement, le système compressible 6 peut comporter un capteur de déplacement 68, à la place ou en plus du capteur de pression 67 précité. Ce capteur de déplacement 68 est apte à mesurer la longueur du dispositif compressible étalonné 66 lors de son allongement ou de sa rétractation, au cours de l'enfoncement du couple tube 2 / tige 1 dans le sol. Cette longueur l est représentative de la résistance du sol, à laquelle elle peut être reliée par une relation de proportionnalité : $R \sim k \times l$, où k est la raideur du dispositif compressible 66 et R la résistance du sol.

Le pénétromètre 100 selon l'invention, muni du système compressible 6, permet donc de mesurer la résistance du sol en continu, au fur et à mesure d'un enfoncement en mode statique du couple tube 2 / tige 1 dans le sol. Avantageusement, le ou les capteur(s) 67,68 comporte(nt) un système d'enregistrement pour collecter les mesures en continu au cours de l'enfoncement. A titre d'exemple, le capteur de pression 67 pourra effectuer une mesure tous les dixièmes secondes ou encore toutes les secondes de la pression dans la deuxième chambre 64.

La configuration de système compressible 6 avec colonne déportée 63 permet d'alléger et de simplifier l'ensemble composé de la cellule de mesure 3 et du train de tube 2 / tige 1, qui doit être maintenu selon l'axe d'enfoncement (parallèle à l'axe du train de tiges). En effet, l'alternative à cette configuration, qui consisterait à positionner le dispositif compressible étalonné 66 directement entre la cellule de mesure 3 et la tige centrale 1, oblige à maintenir ledit dispositif 66 dans l'axe d'enfoncement et augmente l'encombrement du pénétromètre.

De manière avantageuse, la colonne déportée 63 comprend une vis de réglage 69 configurée pour faire varier le volume de la deuxième chambre 64 en se déplaçant dans la colonne déportée 63. Préférentiellement, la vis de réglage 69 est actionnée par un moteur 70 asservi et piloté par un contrôleur électronique. Le déplacement et la vitesse de déplacement de la vis de réglage 69 peuvent ainsi être commandés. Notons que par vis de réglage on entend tout moyen actionné manuellement ou automatiquement, apte à effectuer un mouvement de translation dans la colonne 63 de manière à modifier le volume de la deuxième chambre 64. Par exemple, un piston de vérin pourrait également correspondre à ladite vis de réglage 69.

En faisant varier le volume de la deuxième chambre 64, la vis 69 va plus ou moins comprimer le dispositif compressible étalonné 66 et plus ou moins appliquer de pression sur la tige centrale 1 (via le premier piston 62) du fait de la communication entre les chambres 64,61. La vis de réglage 69 permet ainsi d'adapter la course de la pointe 11, entre une position sortie et sa position effacée, en fonction du massif de réaction utilisé. Le massif de réaction est décrit ci-dessous en liaison avec les moyens d'appui 4 pour l'enfoncement en mode statique du couple tube 2 / tige 1.

Préférentiellement, le pénétromètre 100 statique selon l'invention comprend également des moyens d'appui 4, adaptés pour appliquer une force F à la cellule de mesure 3, et permettant d'effectuer un enfoncement dans le sol en mode statique du couple formé par le tube creux 2 et la tige centrale 1. La force F est appliquée à une partie extérieure 3b de la cellule 3.

Les moyens d'appui pourront en particulier comprendre au moins un vérin hydraulique apte à développer une puissance comprise entre 5 et 15 Tonnes. Une partie 41 du vérin est fixée à la partie extérieure 3b de la cellule 3 et applique à celle-ci la force nécessaire à l'enfoncement continu du couple tube 2/tige 1. Une autre partie 42 du vérin doit être fixé directement ou indirectement à un massif de réaction 200.

Les moyens d'appui 4 pourront comprendre un groupe hydraulique automoteur, pour actionner le vérin 41,42.

Avantageusement, les moyens d'appui 4 sont maintenus par un châssis 5. Le châssis 5 est muni d'au moins un élément de liaison mécanique destiné à être connecté à un massif de réaction 200. Cet élément de liaison mécanique pourra par exemple consister en une pince hydraulique ou mécanique, ou encore un étau de même type. Le fait que le châssis 5 soit équipé d'un tel élément de liaison mécanique le rend connectable à tout genre de massif de réaction 200 : grue, pelle mécanique, poids lourd, etc.

Préférentiellement, le pénétromètre 100 comprend des moyens supplémentaires d'enfoncement 8 aptes à appliquer au tube creux 2, une vibration à une fréquence déterminée. Nous verrons plus loin que cette vibration, appliquée temporairement au cours de l'enfoncement quand un sol à forte résistance est rencontré, aide à poursuivre l'enfoncement en mode statique, sans nécessiter de massif de réaction 200 surdimensionné ou l'utilisation d'une assistance dynamique qui empêche toute mesure précise de la résistance du sol.

De manière avantageuse, ces moyens supplémentaires 8 sont couplés à une partie interne 3a de la cellule 3 qui est solidaire du tube creux 2. La vibration appliquée à la partie interne 3a est ainsi transmise au tube creux 2.

La première chambre 61 est formée dans cette partie interne 3a. Il est prévu que la partie interne 3a puisse être mobile selon l'axe d'enfoncement dans la partie externe 3b de la cellule de mesure 3. Lorsque la vibration est appliquée à la partie interne 3a, cette dernière est ainsi libre d'osciller en limitant la transmission de la vibration à la partie externe 3b solidaire des moyens d'appui 4.

Dans le premier mode de réalisation illustré en figure 1, le tuyau flexible 614a est directement connecté à la première chambre 61 via un premier orifice prévu dans la partie interne 3a. Un deuxième orifice aménagé dans la partie externe 3b de la cellule 3 permet au tuyau flexible 614a de relier la deuxième chambre 64 et laissera la latitude suffisante pour accommoder l'oscillation de la partie interne 3a lorsqu'une vibration lui est appliquée.

Dans le deuxième mode de réalisation illustré en figure 2, des joints 3c sont disposés entre la partie interne 3a et la partie externe 3b de la cellule 3 et définissent un espace annulaire 3d étanche entre les deux parties 3a,3b. Un premier orifice dans la partie interne 3a établit une communication fluidique entre la première chambre 61 et l'espace annulaire 3d ; un deuxième orifice dans la partie externe 3b établit une communication fluidique entre l'espace annulaire 3d et le conduit 614b, qui connecté audit deuxième orifice, assure la communication avec la deuxième chambre 64. Cette configuration de connexion fluidique évite que le conduit rigide 614b subisse les vibrations appliquées à la partie interne 3a par les moyens supplémentaires 8, susceptibles de le dégrader. Une telle configuration pourrait également être mise en œuvre dans le mode de réalisation illustré en figure 1, dans lequel le système compressible 6 comprend un conduit flexible 614a, mais présente moins d'intérêt, le conduit souple 614a étant capable d'encaisser les vibrations.

Le pénétromètre selon l'invention peut donc recourir à une assistance vibro-dynamique, tout en conservant un enfoncement en mode statique et une mesure via le système compressible 6 continue et précise, représentative de la dureté du sol.

A titre d'exemple, les moyens supplémentaires 8 d'enfoncement sont composés par au moins un dispositif hydraulique, pneumatique ou électrique (ou autre source d'énergie) de vibro-fonçage, procurant une énergie mécanique par impulsion, à une fréquence déterminée. Préférentiellement, la fréquence déterminée de la vibration est comprise entre 35 et 100 Hz. Et l'énergie unitaire, c'est-à-dire à chaque impulsion ou coup, développée par les moyens supplémentaires d'enfoncement est comprise entre 25 et 400 Joules.

Il est à noter que la configuration de système compressible 6 avec colonne déportée 63 permet de fiabiliser ledit système compressible 6, dont les parties mécaniques (notamment le dispositif compressible étalonné 66) ne sont pas directement soumises à la vibration susceptible d'être appliquée pour l'enfoncement du train de tubes 2 / tige 1, puisqu'elles sont disposées dans la colonne déportée 63 (ou la deuxième colonne déportée 63').

Le pénétromètre 100 peut en outre comprendre un contrôleur électronique apte à analyser les mesures issues du ou des capteurs 67,68, à actionner la vis de réglage 69 (par exemple, via le moteur 70), et à activer ou désactiver les moyens supplémentaires d'enfoncement 8, selon des critères programmés ou définis par un opérateur au cours de l'essai.

Mesure de la résistance d'un sol en mode statique

Le pénétromètre 100 selon la présente invention peut être utilisé pour la mesure de la résistance à la pénétration d'un sol, en particulier selon le procédé de mesure en mode statique décrit ci-après.

Pour réaliser un essai, le châssis 5 du pénétromètre 100 est connecté à un massif de réaction 200. La force d'appui maximale qui pourra être appliquée à la cellule de mesure 3 pour l'enfoncement du couple tube 2 / tige 1 est définie par la masse dudit massif de réaction 200. Si, au cours de l'essai, la pointe 11 rencontre un sol dont la résistance excède la force d'appui maximale, le pénétromètre 100 est apte à recourir à une assistance vibro-dynamique grâce aux moyens supplémentaires d'enfoncement 8. Pour éviter d'endommager la tige 1, il est important que la pointe 11 soit en position effacée (c'est-à-dire en appui contre le tube creux 2) lorsque l'assistance vibro-dynamique est mise en œuvre.

La vis de réglage 69 du système compressible 6 permet d'adapter la course de la pointe 11 au massif de réaction 200, de sorte que ladite pointe 11 se trouve en position effacée lorsque la résistance du sol atteint la force d'appui maximale.

A la suite de ce réglage, le procédé de mesure comprend en premier lieu l'enfoncement dans le sol en mode statique (enfoncement continu et régulier) du couple formé par le tube creux 2 et la tige centrale 1, à une vitesse d'enfoncement donnée. La vitesse d'enfoncement en mode statique est comprise entre 1 et 5 cm/s ; avantageusement, pour respecter des normes de mesures en vigueur, la vitesse d'enfoncement est de l'ordre de 2cm/s.

Au cours de cet enfoncement, le procédé prévoit la mesure, par le capteur de pression 67, de la pression dans la deuxième chambre 64 du système compressible 6. Cette mesure de pression permet de remonter à la résistance en pointe du sol (Qc), pour des profondeurs successives d'enfoncement dans le sol.

Lorsque la pointe de mesure 11 rencontre un sol très résistant, pour lequel la force d'appui maximale est atteinte, la pointe 11 se retrouve dans sa position effacée (c'est-à-dire en appui contre le tube creux 2) . Le procédé prévoit alors l'application par les moyens supplémentaires d'enfoncement 8, d'un vibro-fonçage à une fréquence déterminée. Comme évoqué précédemment, la fréquence déterminée est préférentiellement comprise entre 35 et 100 Hz, et l'énergie unitaire (par impulsion) est comprise entre 25 et 400 Joules. La vibration appliquée, transmise au tube creux 2, soit directement, soit par l'intermédiaire de la partie interne 3a de la cellule de mesure 3, permet d'aider au franchissement des couches de sol particulièrement dures.

L'application d'une vibration grâce aux moyens supplémentaires d'enfoncement permet de traverser cette couche, jusqu'à atteindre une couche de dureté moindre. A ce moment-là, le dispositif compressible 66 va s'allonger, la pointe de mesure 11 en bout de la tige 1 rencontrant une plus faible résistance. Les moyens supplémentaires d'enfoncement 8 sont alors désactivés et l'enfoncement en mode statique reprend.

Le procédé selon l'invention permet ainsi de mesurer la résistance du sol en continu et sans interruption car le franchissement des couches dures en profondeur se fait progressivement, par vibro-fonçage. Dès que la dureté du sol diminue, la mesure par le ou les capteur (s) 67,68 du système compressible 6 permet d'obtenir une valeur de résistance du sol. On ne perd ainsi aucune information sur les caractéristiques du sol, qui sont mesurées en continu via les variations de pression dans la deuxième chambre 64 du système compressible 6 et/ou via les variations de longueur du dispositif compressible étalonné 66.

Avantageusement, un contrôleur électronique est configuré pour enregistrer les mesures issues du ou des capteurs (de pression 67 et/ou de déplacement 68) et pour activer ou désactiver les moyens supplémentaires d'enfoncement 8. Il peut également être configuré pour commander les moyens d'appui 4 et asservir la force F appliquée à la cellule de mesure 3, à la vitesse d'enfoncement du couple tube 2 / tige 1 dans le sol.

Notons que le premier et le deuxième mode de mise en œuvre du pénétromètre 100 (figures 1 et 2) sont adaptés à la réalisation du présent procédé de mesure de la résistance d'un sol en mode statique.

## Évaluation du caractère liquéfiable d'un sol

Le pénétromètre 100 selon la présente invention peut également être utilisé pour l'évaluation du caractère liquéfiable d'un sol, en particulier en appliquant le procédé décrit ci-après.

Le procédé comprend une première étape a) consistant à enfoncer dans le sol le couple tube 2 / tige 1 pour amener la pointe de mesure 11 à une profondeur donnée pour l'investigation du caractère liquéfiable. Les moyens d'appui 4, par l'intermédiaire de la cellule 3, permettent de réaliser cet enfoncement à la profondeur donnée.

Après que la pointe de mesure 11 a atteint la profondeur donnée, il est important qu'elle ne soit pas dans sa position complètement sortie : la course de la pointe 11 pourra à ce moment-là être adaptée via la vis de réglage 69 du système compressible 6 (en faisant varier le volume de la deuxième chambre 64).

A partir de là, le dispositif compressible étalonné 66 est bridé, de manière à demeurer à longueur constante pour les étapes suivantes du procédé.

Une deuxième étape b) du procédé consiste à induire, à partir de la profondeur donnée, un premier enfoncement contrôlé de la pointe de mesure 11 dans le sol. Le dispositif compressible étalonné 66 étant bridé, l'actionnement de la vis de réglage 69 par le moteur 70 asservi et piloté permet de réduire le volume de la deuxième chambre 64 et ainsi d'augmenter la pression contre le premier piston 62 pour induire un premier déplacement de la tige 1 (correspondant au premier enfoncement contrôlé de la pointe 11). Par exemple, le premier enfoncement est de 10mm et est réalisé à une vitesse constante de 2cm/s.

La pression appliquée (pression mesurée via le capteur de pression 67) pour effectuer ledit premier enfoncement est représentative de la résistance du sol et donne une résistance de pointe $Q_c^{2cm/s}$. Préférentiellement, après le premier enfoncement, la pression dans la deuxième chambre 64, à l'arrêt (représentative de la résistance de pointe à l'arrêt $Q_c^{arrêt}$) est également mesurée.

Les valeurs $Q_c^{2cm/s}$ et $Q_c^{arrêt}$ extraites des mesures à l'étape b) constituent le point de départ de l'essai d'évaluation du caractère liquéfiable de la couche de sol investiguée.

Une troisième étape c) du procédé consiste à appliquer une vibration à basse fréquence à la deuxième extrémité 12 de la tige centrale 1 et simultanément à lui faire effectuer un deuxième déplacement induisant un deuxième enfoncement contrôlé de la pointe 11 dans le sol. Une telle vibration basse fréquence est appliquée par l'intermédiaire de la vis de réglage 69, qui en diminuant progressivement le volume de la deuxième chambre 64 par des mouvements d'aller-retour à la fréquence choisie, va appliquer la vibration et générer le deuxième enfoncement.

La basse fréquence choisie est comprise entre 1 et 5 Hertz, fréquences caractéristiques des séismes. L'objectif ici est d'appliquer localement des contraintes susceptibles de modifier les propriétés de la couche de sol, comme pourrait le faire un tremblement de terre. Le deuxième enfoncement de la pointe 11 dans la couche de sol s'effectue alors qu'elle est mise sous contrainte vibratoire. Par exemple, le deuxième enfoncement pourra être de 30mm.

La pression appliquée pour effectuer le deuxième enfoncement est représentative de la résistance de pointe $Q_c$ au cours de ce deuxième enfoncement contrôlé.

Avantageusement, la vitesse du deuxième enfoncement est ajustée de manière à maintenir la pression appliquée sensiblement constante, grâce à un asservissement du moteur actionnant la vis de réglage 69. Le deuxième enfoncement est donc avantageusement réalisé à charge (force appliquée) constante. En particulier, on vise une force appliquée sensiblement égale à la valeur de force appliquée représentative de la résistance de pointe $Q_c^{2cm/s}$ mesurée à l'étape b). La vitesse du deuxième enfoncement est donc automatiquement augmentée ou diminuée en fonction de la pression mesurée au cours de l'étape c), dans l'objectif de maintenir celle-ci sensiblement constante. La vitesse à laquelle le deuxième enfoncement est effectué, à force constante, donne une indication importante sur le caractère brutal de la perte de résistance de la couche de sol sous sollicitation vibratoire.

Plusieurs cas de figures peuvent se présenter, selon les propriétés de la couche de sol analysée. Les propriétés de résistance de la couche de sol peuvent évoluer très lentement sous la sollicitation vibratoire : le deuxième enfoncement s'effectue alors à une faible vitesse. Dans un tel cas de figure, il apparaît que la couche de sol ne se modifie pas brusquement avec une contrainte vibratoire. Selon un autre cas de figure, la couche de sol peut perdre très rapidement sa résistance sous la sollicitation vibratoire : le deuxième enfoncement s'effectue alors à une vitesse élevée. Les risques en cas de séisme dans l'un et l'autre cas de figures évoqués sont clairement différents : les précautions et spécifications sur les fondations d'ouvrages construits pourront être adaptées à chaque cas.

Lorsque le deuxième enfoncement contrôlé est terminé la vibration est arrêtée.

La quatrième étape d) du procédé prévoit d'actionner la vis de réglage 67 de manière à provoquer un troisième enfoncement contrôlé de la pointe de mesure 11 dans le sol, préférentiellement à vitesse constante.

Par exemple, le troisième enfoncement pourra être de 10mm, réalisé à une vitesse de 2cm/s.

Au cours de l'étape d), en l'absence de sollicitation vibratoire, la couche de sol peut voir sa résistance évoluer de différentes manières, selon les caractéristiques de ladite couche.

Selon un premier comportement, une couche ayant rapidement perdu sa résistance sous sollicitation vibratoire pourra, en l'absence de cette dernière, retrouver sa résistance initiale $Q_c^{2cm/s}$ : c'est un comportement de sol liquéfiable.

Selon un deuxième comportement, une couche ayant rapidement perdu sa résistance sous sollicitation vibratoire pourra, en l'absence de cette dernière, revenir lors du troisième enfoncement contrôlé à une résistance $Q_c$ inférieure à sa résistance initiale $Q_c^{2cm/s}$ : cela peut traduire un phénomène de type grande déformation, les propriétés de résistance de la couche de sol ayant été irréversiblement modifiées par la vibration.

Enfin, selon un troisième comportement, une couche ayant rapidement perdu sa résistance sous sollicitation vibratoire pourra, en l'absence de cette dernière, revenir lors du troisième enfoncement contrôlé à une résistance $Q_c$ supérieure à sa résistance initiale $Q_c^{2cm/s}$ : cela peut traduire un phénomène de densification, la résistance de la couche de sol ayant été renforcée par la vibration.

Les étapes a) à d) pourront être répétées pour d'autres profondeurs données d'investigation, de manière à analyser des couches de sol successives incluses dans une zone suspecte de plus ou moins grande épaisseur.

Le procédé précité et le pénétromètre 100 selon l'invention permettent ainsi d'évaluer le caractère liquéfiable de la couche de sol analysée, à partir de la vitesse de réalisation du deuxième enfoncement. La diminution plus ou moins brutale de la résistance du sol sous sollicitation vibratoire est un critère clé du caractère liquéfiable. On peut également tirer des informations importantes sur les propriétés de portance de la couche de sol analysée, à la suite d'une sollicitation vibratoire, permettant d'anticiper de potentielles modifications irréversibles de la résistance du sol.

Notons que le pénétromètre 100 selon le deuxième mode de mise en œuvre (figure 2) sera préférentiellement utilisé pour réaliser ce procédé, la communication fluidique rigide et courte entre la deuxième chambre 64 et la première chambre 61 étant plus adaptée à une application efficace de la sollicitation cyclique (vibration basse fréquence) au piston 62 (en liaison avec la tige 1), par la vis de réglage 69.

Mesure des propriétés élasto-plastique d'un sol

Le pénétromètre 100 selon la présente invention peut également être utilisé pour la mesure des propriétés élasto-plastiques d'un sol, notamment en appliquant le procédé décrit ci-après.

Le procédé comprend tout d'abord une première étape a) consistant à enfoncer dans le sol le couple formé par le tube creux 2 et la tige centrale 1 pour amener la pointe de mesure 11 à une profondeur souhaitée pour la mesure des propriétés élasto-plastiques du sol. Arrivée à ladite profondeur, la pointe de mesure 11 est effacée, c'est-à-dire placée en butée contre le tube creux 2, au moyen de la vis de réglage 69 (c'est-à-dire en faisant varier le volume de la deuxième chambre 64).

A partir de là, le dispositif compressible étalonné 66 est bridé, de manière à demeurer à longueur constante pour les étapes suivantes du procédé.

Le procédé comprend une deuxième étape b) au cours de laquelle une première force $F_1$ constante est appliquée à une deuxième extrémité 12 de la tige 1, pendant une durée déterminée t. Rappelons qu'une force peut être appliquée sur le premier piston 62 en liaison avec la tige 1, par l'intermédiaire de la vis de réglage 69 qui peut faire varier la pression dans la deuxième chambre 64 et dans la première chambre 61 (les deux chambres 61,64 étant en communication fluidique).

La force constante $F_1$ appliquée est transmise par la tige 1 à la pointe de mesure 11, qui va plus ou moins s'enfoncer dans le sol, selon les caractéristiques mécaniques de celui-ci.

La première force $F_1$ appliquée pourra être comprise entre 100N et 500N ; en considérant une section de 10 cm2 de la pointe de mesure 11, cela correspond à une contrainte appliquée comprise entre 1 et 5 bars.

Dans une troisième étape c) du procédé, on mesure un premier déplacement final $D_1^t$ , associé à l'enfoncement de la pointe de mesure 11 dans le sol, au bout de la durée déterminée t. On qualifie ici de « final », un déplacement correspondant au déplacement relevé à la fin de la durée déterminée t.

Le procédé prévoit ensuite la réitération des étapes b) et c), avec des forces appliquées croissantes, pour former une courbe représentant le déplacement final en fonction de la force appliquée. Ainsi, une deuxième force $F_2$ constante, supérieure à $F_1$, est appliquée à la deuxième extrémité 12 de la tige 1, pendant la durée déterminée t. Une mesure est faite du deuxième déplacement final $D_2^t$ , associé à l'enfoncement de la pointe de mesure 11 dans le sol, au bout de la durée déterminée t. A noter que ce deuxième déplacement final $D_2^t$ correspond au cumul du premier déplacement final $D_1^t$ et du déplacement additionnel provoqué par l'application de la deuxième force $F_2$. Une troisième force $F_3$ constante, supérieure à $F_2$, est ensuite appliquée, toujours pendant la durée déterminée t, puis une mesure du troisième déplacement final $D_3^t$ est réalisée au bout de ladite durée t, et ainsi de suite.

Avantageusement, l'incrément entre deux forces appliquées successives est compris entre 100 et 500N.

La durée déterminée t pourra varier entre 15 et 600 secondes ; elle est préférentiellement définie à t = 60s car cette durée rejoint l'approche méthodologique des essais pressiométriques connus.

La séquence d'application d'une force et de mesure du déplacement final est répétée n fois (application d'une énième force $F_n$ constante, supérieure à $F_{n-1}$, et mesure d'un énième déplacement final $D_n^t$ ) jusqu'à atteindre la contrainte à la rupture du sol Qc. La contrainte à la rupture du sol va correspondre à une force $F_n$ engendrant un déplacement final $D_n^t$ maximal dû à un important enfoncement de la pointe de mesure 11. Rappelons que la force $F_n$ est reliée à la contrainte par la relation : Qc = $F_n$ / S, S étant la surface de la section plane de la pointe de mesure 11 (par exemple 10cm2).

En pratique, le déplacement maximal représentatif de la rupture du sol est défini à environ 5 cm (amplitude de sortie de la pointe 11 par rapport à sa position effacée contre le tube creux 2).

A l'issue des étapes précédentes, on peut tracer une courbe représentant le déplacement final en fonction de la force appliquée. La courbe est formée d'un premier domaine correspondant à des déformations élastiques du sol. Elle est formée, après un point d'inflexion, d'un deuxième domaine correspondant à des déformations plastiques du sol, jusqu'à atteindre le point de rupture (Qc).

Le procédé comprend ensuite l'extraction du module de déformation M du sol à partir de la pente de la courbe déplacement final / force, dans le premier domaine de déformation élastique situé avant le point d'inflexion.

La pente p de la courbe dans le premier domaine élastique E s'exprime :

$$p = \frac{(D_3^t - D_1^t)}{(F_3 - F_1)}$$ (notons que $D_3^t$ et $F_3$ sont donné ici à titre d'exemple, le principe étant d'utiliser un couple déplacement final / force, dans le domaine élastique, permettant de calculer de manière précise la pente dudit domaine)

Le module de déformation M, en MPa/m, est calculé à partir de l'expression : $$M = \frac{1}{S \times p}$$ avec p la pente du premier domaine élastique et S la surface de la section plane de la pointe de mesure 11. Le module ainsi extrait permet la modélisation du tassement du sol sous charge.

Le procédé permet également de déterminer la contrainte de fluage (Qf), limite élastique du sol. En effet, partant de la courbe déplacement final / force, la force $F_f$ correspondant au point d'inflexion, traduit ladite contrainte de fluage Qf.

La contrainte de fluage s'exprime :

$$Q_f = \frac{F_f}{S}$$ avec $F_f$ la force correspondant au point d'inflexion et S la surface de la section plane de la pointe de mesure 11.

A partir de la contrainte de fluage Qf, on pourrait estimer, à titre sécuritaire, une valeur de contrainte admissible Qa à ½ Qf, qui placerait la contrainte admissible Qa en partie médiane du domaine élastique E. Rappelons que la contrainte admissible Qa est utilisée pour le dimensionnement des fondations de l'ouvrage.

Selon une autre approche, en fonction du tassement admissible du projet d'ouvrage, on pourrait attribuer à la contrainte admissible Qa, une valeur, toujours inférieure à la contrainte de fluage Qf, située dans le premier domaine élastique, et correspondant sur la courbe, à un déplacement égal au tassement admissible connu. Rappelons que traditionnellement, la contrainte admissible Qa est évaluée sur la base d'une fraction de la contrainte à la rupture Qc : Qa = Qc/10 ; le coefficient 10 étant établi semi-empiriquement et de manière extrêmement sécuritaire. Le présent procédé donne une valeur de la contrainte admissible Qa basée sur la gestion du tassement admissible vis-à-vis de l'ouvrage, ce qui la rend beaucoup plus pertinente.

Le pénétromètre 100 selon l'invention permet donc une mesure in situ pour reconstituer directement la relation d'action / réaction d'un ensemble fondations / sol en donnant accès au comportement effectif sous charge du sol : portance, tassements, fluage dans le temps.

Notons que le pénétromètre 100 selon le deuxième mode de mise en œuvre (figure 2) sera préférentiellement utilisé pour ce ces mesures élasto-plastiques, la communication fluidique rigide et courte entre la deuxième chambre 64 et la première chambre 61 étant plus adaptée à une application efficace des séquences force/déplacement au piston 62 (en liaison avec la tige 1) par la vis de réglage 69.

## Revendications

1. Pénétromètre (100) comprenant :

   - Au moins une tige centrale (1) terminée à une première extrémité par une pointe de mesure (11) ;
   - Au moins un tube creux (2) entourant la tige centrale (1), cette dernière étant apte à coulisser à l'intérieur du tube creux (2) ;
   - Une cellule de mesure (3) en contact avec le tube creux (2), destinée à transmettre une force appliquée par des moyens d'appui (4), de manière à provoquer un enfoncement en mode statique dans le sol du tube creux (2) et de la tige centrale (1) ; ledit pénétromètre (100) comprenant un système compressible (6) assurant une liaison élastique entre la cellule de mesure (3) et une seconde extrémité (12) de la tige centrale (1), ledit pénétromètre (100) étant **caractérisé en ce que** ledit système compressible (6) comporte:

   - Une première chambre (61) à huile formée dans la cellule de mesure (3), et un premier piston (62) solidaire d'une seconde extrémité (12) de la tige centrale (1) et apte à coulisser dans la première chambre (61) ;
   - Au moins une colonne (63) déportée par rapport à l'axe de la tige centrale (1), qui comprend une deuxième chambre (64) à huile en communication fluidique avec la première chambre (61), un deuxième piston (65) apte à coulisser dans la deuxième chambre (64) et un dispositif compressible étalonné (66) en contact avec le deuxième piston (65) ;
   - Un capteur de pression (67) relié à la deuxième chambre et/ou un capteur de déplacement (68) apte à mesurer la longueur du dispositif compressible étalonné (66).

2. Pénétromètre selon la revendication précédente, dans lequel la communication fluidique entre la première chambre (61) et la deuxième chambre (64) est faite au moyen d'un tuyau flexible (614a).

3. Pénétromètre selon la revendication 1, dans lequel la communication fluidique entre la première chambre (61) et la deuxième chambre (64) est faite au moyen d'un conduit rigide (614b).

4. Pénétromètre selon la revendication précédente, dans lequel :

   - la cellule de mesure (3) comporte une partie interne (3a), une partie externe (3b) et un espace annulaire étanche (3d) entre la partie interne (3a) et la partie externe (3b),
   - la première chambre (61) est formée dans la partie interne (3a) et est apte à communiquer avec l'espace annulaire (3d) via un premier orifice,
   - le conduit rigide (614b) est fixé à la partie externe (3b) qui comprend un deuxième orifice pour communiquer avec l'espace annulaire (3d).

5. Pénétromètre selon l'une des revendications précédentes, dans lequel la colonne déportée (63) comprend une vis de réglage (69) configurée pour faire varier le volume de la deuxième chambre (64) en se déplaçant dans la colonne déportée (63).

6. Pénétromètre selon la revendication précédente, dans lequel la vis de réglage (69) est actionnée par un moteur (70) asservi et piloté par un contrôleur électronique.

7. Pénétromètre selon l'une des revendications précédentes, comprenant des moyens supplémentaires d'enfoncement (8) aptes à appliquer au tube creux (2), une vibration à une fréquence déterminée.

8. Pénétromètre selon la revendication précédente, dans lequel les moyens supplémentaires d'enfoncement (8) sont composés par au moins un dispositif hydraulique, pneumatique ou électrique de vibrofonçage.

9.  Pénétromètre selon l'une des revendications précédentes, dans lequel le dispositif compressible (66) est formé par un ressort étalonné ou par une pluralité de rondelles Belleville.

10. Pénétromètre selon l'une des revendications précédentes, comprenant les moyens d'appui (4) aptes à appliquer une force à la cellule de mesure (3), pour effectuer un enfoncement dans le sol en mode statique du tube creux (2) et de la tige centrale (1).

11. Pénétromètre selon l'une des revendications précédentes, comprenant un châssis (5) muni d'au moins un élément de liaison mécanique destiné à être connecté à un massif de réaction (200).

12. Pénétromètre selon la revendication précédente, dans lequel la course de la pointe de mesure (11) par rapport à une extrémité du tube creux (2) est définie par la variation du volume de la deuxième chambre (64) et adaptée au massif de réaction (200).

13. Utilisation du pénétromètre (100) selon l'une des revendications précédentes, pour la mesure de la résistance à la pénétration d'un sol; ou pour l'évaluation du caractère liquéfiable d'un sol ou pour la mesure des propriétés élasto-plastiques d'un sol.


**Patentansprüche**

1.  Penetrometer (100), umfassend:

    - mindestens einen zentralen Stab (1), der an einem ersten Ende mit einer Messspitze (11) endet;
    - mindestens ein hohles Rohr (2), das den zentralen Stab (1) umgibt, wobei dieser geeignet ist, in dem hohlen Rohr (2) zu gleiten;
    - eine Messzelle (3), die mit dem hohlen Rohr (2) in Kontakt steht und dazu bestimmt ist, eine von einem Stützmittel (4) aufgebrachte Kraft zu übertragen, so dass das hohle Rohr (2) und der zentrale Stab (1) im statischen Modus in den Boden vorgetrieben werden;

    wobei das Penetrometer (100) ein komprimierbares System (6) umfasst, das eine elastische Verbindung zwischen der Messzelle (3) und einem zweiten Ende (12) des zentralen Stabs (1) herstellt, wobei das Penetrometer (100) **dadurch gekennzeichnet ist, dass** das komprimierbare System (6) enthält:

    - eine erste Ölkammer (61), die in der Messzelle (3) ausgebildet ist, und einen ersten Kolben (62), der mit einem zweiten Ende (12) des zentralen Stabs (1) fest verbunden ist und geeignet ist, in der ersten Kammer (61) zu gleiten;

    - mindestens eine von der Achse des zentralen Stabs (1) versetzte Säule (63), die eine zweite Ölkammer (64) in Fluidverbindung mit der ersten Kammer (61), einen zweiten Kolben (65), der geeignet ist, in der zweiten Kammer (64) zu gleiten, und eine kalibrierte komprimierbare Vorrichtung (66) in Kontakt mit dem zweiten Kolben (65) umfasst;
    - einen Drucksensor (67), der mit der zweiten Kammer verknüpft ist, und/oder einen Wegsensor (68), der die Länge der kalibrierten komprimierbaren Vorrichtung (66) messen kann.

2.  Penetrometer nach dem vorhergehenden Anspruch, wobei die Fluidverbindung zwischen der ersten Kammer (61) und der zweiten Kammer (64) mittels eines flexiblen Schlauchs (614a) erfolgt.

3.  Penetrometer nach Anspruch 1, wobei die Fluidverbindung zwischen der ersten Kammer (61) und der zweiten Kammer (64) mittels einer starren Leitung (614b) erfolgt.

4.  Penetrometer nach dem vorhergehenden Anspruch, bei dem:

    - die Messzelle (3) einen inneren Teil (3a), einen äußeren Teil (3b) und einen abgedichteten ringförmigen Raum (3d) zwischen dem inneren Teil (3a) und dem äußeren Teil (3b) enthält,
    - die erste Kammer (61) im inneren Teil (3a) ausgebildet ist und geeignet ist, über eine erste Öffnung mit dem ringförmigen Raum (3d) in Verbindung zu treten,
    - die starre Leitung (614b) an dem äußeren Teil (3b) befestigt ist, der eine zweite Öffnung zur Verbindung mit dem ringförmigen Raum (3d) umfasst.

5.  Penetrometer nach einem der vorhergehenden Ansprüche, wobei die versetzte Säule (63) eine Einstellschraube (69) umfasst, die so konfiguriert ist, dass sie das Volumen der zweiten Kammer (64) durch Bewegung in der versetzten Säule (63) verändert.

6.  Penetrometer nach dem vorhergehenden Anspruch, wobei die Einstellschraube (69) von einem servoangetriebenen Motor (70) betätigt und von einem elektronischen Regler gesteuert wird.

7.  Penetrometer nach einem der vorhergehenden Ansprüche, der zusätzliche Vortriebsmittel (8) umfasst, die geeignet sind, eine Vibration einer bestimmten Frequenz auf das hohle Rohr (2) aufzubringen.

8.  Penetrometer nach dem vorhergehenden Anspruch, wobei die zusätzlichen Vortriebsmittel (8) aus mindestens einer hydraulischen, pneumatischen oder elektrischen Vibrationstriebvorrichtung bestehen.

9. Penetrometer nach einem der vorhergehenden Ansprüche, wobei die komprimierbare Vorrichtung (66) durch eine kalibrierte Feder oder durch mehrere Tellerfedern ausgebildet wird.

10. Penetrometer nach einem der vorhergehenden Ansprüche, umfassend Stützmittel (4), die geeignet sind, eine Kraft auf die Messzelle (3) aufzubringen, um einen statischen Vortrieb des hohlen Rohrs (2) und des zentralen Stabs (1) in den Boden zu bewirken.

11. Penetrometer nach einem der vorhergehenden Ansprüche, umfassend einen Rahmen (5), der mit mindestens einem mechanischen Verbindungselement versehen ist, das dazu bestimmt ist, an eine Reaktionsmasse (200) angeschlossen zu werden.

12. Penetrometer nach dem vorhergehenden Anspruch, wobei der Hub der Messspitze (11) relativ zu einem Ende des hohlen Rohrs (2) durch die Veränderung des Volumens der zweiten Kammer (64) definiert und an die Reaktionsmasse (200) angepasst ist.

13. Verwendung des Penetrometers (100) nach einem der vorhergehenden Ansprüche zur Messung des Eindringwiderstandes eines Bodens; oder zur Beurteilung der Verflüssigbarkeit eines Bodens oder zur Messung der elasto-plastischen Eigenschaften eines Bodens.

**Claims**

1. Penetrometer (100), comprising:

   - at least one central rod (1), a first end of which finishes in a measuring tip (11);
   - at least one hollow tube (2) surrounding the central rod (1), said rod being capable of sliding inside the hollow tube (2);
   - a measurement cell (3) which is in contact with the hollow tube (2) and intended to transmit a force applied by bearing means (4) so as to drive the hollow tube (2) and the central rod (1) into the soil in static mode;

   said penetrometer (100) comprising a compressible system (6) which provides an elastic link between the measurement cell (3) and a second end (12) of the central rod (1), said penetrometer (100) being **characterized in that** said compressible system (6) comprises:

   - a first oil chamber (61) which is formed in the measurement cell (3) and a first plunger (62) which is rigidly connected to a second end (12) of the central rod (1) and capable of sliding in the first chamber (61);
   - at least one column (63) which is offset with respect to the axis of the central rod (1) and comprises a second oil chamber (64) in fluid communication with the first chamber (61), a second plunger (65) capable of sliding in the second chamber (64), and a calibrated compressible device (66) in contact with the second plunger (65);
   - a pressure sensor (67) connected to the second chamber and/or a displacement sensor (68) capable of measuring the length of the calibrated compressible device (66).

2. Penetrometer according to the preceding claim, wherein the fluid communication between the first chamber (61) and the second chamber (64) is established by means of a flexible pipe (614a).

3. Penetrometer according to claim 1, wherein the fluid communication between the first chamber (61) and the second chamber (64) is established by means of a rigid conduit (614b).

4. Penetrometer according to the preceding claim, wherein:

   - the measurement cell (3) comprises an internal part (3a), an external part (3b), and a sealed annular space (3d) between the internal part (3a) and the external part (3b),
   - the first chamber (61) is formed in the internal part (3a) and is capable of communicating with the annular space (3d) via a first opening,
   - the rigid conduit (614b) is fixed to the external part (3b), which comprises a second opening to communicate with the annular space (3d).

5. Penetrometer according to any of the preceding claims, wherein the offset column (63) comprises an adjusting screw (69) designed to vary the volume of the second chamber (64) by moving in the offset column (63).

6. Penetrometer according to the preceding claim, wherein the adjusting screw (69) is actuated by a servo motor (70) and controlled by an electronic controller.

7. Penetrometer according to any of the preceding claims, comprising additional driving means (8) capable of applying a vibration at a determined frequency to the hollow tube (2).

8. Penetrometer according to the preceding claim, wherein the additional driving means (8) consist of at least one hydraulic, pneumatic, or electric vibratory driving device.

9. Penetrometer according to any of the preceding claims, wherein the compressible device (66) is formed by a calibrated spring or by a plurality of Belleville washers.

10. Penetrometer according to any of the preceding claims, comprising the bearing means (4) capable of applying a force to the measurement cell (3) to drive the hollow tube (2) and the central rod (1) into the soil in static mode.

11. Penetrometer according to any of the preceding claims, comprising a frame (5) provided with at least one mechanical link element intended to be connected to a counterweight (200).

12. Penetrometer according to the preceding claim, wherein the travel of the measuring tip (11) relative to one end of the hollow tube (2) is defined by the variation in the volume of the second chamber (64) and adapted to the counterweight (200).

13. Use of the penetrometer (100) according to any of the preceding claims for measuring soil penetration resistance or for evaluating the liquefiability of soil or for measuring the elastoplastic properties of soil.

[Fig. 1]

[Fig. 2]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2584186

- EP 2535460 A